# EUROPÄISCHE PATENTSCHRIFT

(11) **EP 0 814 075 B1**
(45) Veröffentlichungstag und Bekanntmachung des Hinweises auf die Patenterteilung: **12.09.2001**
(21) Anmeldenummer: 97109361.2
(22) Anmeldetag: 10.06.1997
(51) Int. Cl.: C07C 209/60

(54) **Verfahren zur Herstellung von Aminen aus Olefinen**
Process for the preparation of amines from olefines
Procédé pour la préparation d'amines à partir d'oléfines

(30) Priorität: 18.06.1996 DE 19624206
(43) Veröffentlichungstag der Anmeldung: 29.12.1997
(73) Patentinhaber: BASF Aktiengesellschaft, 67063 Ludwigshafen (DE)
(72) Erfinder: Eller, Karsten, Dr., 67059 Ludwigshafen (DE); Kummer, Rudolf, Dr., 67227 Frankenthal (DE); Hesse, Michael, Dr., 67549 Worms (DE)

(56) Entgegenhaltungen:
- EP-A- 0 699 653
- S. KLEIN ET A.: "Amorphous microporous Titania-Silica misxed oxides: Preparation, characterization, and catalytic redox properties", JOURNAL OF CATALYSIS, , 1996, Band 163, Nr. , Seiten 476 - 488
- : "Ullman's Encyclopedia of Industrial Chemistry; 5th Edition; Vol. A5, p. 313, 347-350", , ,

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drücken in Gegenwart eines Heterogenkatalysators.

Eine Übersicht über die Methoden zur Aminierung von Olefinen wird in "Functionalisation of Alkenes: Catalytic Amination of Monoolefins", J.J. Brunet et al., J. Mol. Catal., 49 (1989), Seiten 235 bis 259 gegeben.

Grundsätzlich gibt es zwei Katalysemechanismen. Das Olefin wird über einen Metallkomplex koordiniert. Diese aktivierte Spezies kann von dem nucleophilen Amin angegriffen werden und ein höher aminiertes Produkt bilden. Das Amin kann an Säurezentren oder an Metallzentren (via Metallamide) chemisorbiert werden und so aktiviert mit dem Olefin umgesetzt werden.

Gut geeignete Katalysatoren sind Zeolithe. Sie zeichnen sich durch eine hohe Anzahl an katalytisch aktiven Zentren aus, kombiniert mit einer großen Oberfläche. Die beschriebenen Zeolithe unterscheiden sich im Typ und in der Nachbehandlung (z.B. thermische Behandlung, Dealuminierung, Säurebehandlung, Metallioneneintausch, etc.). Beispiele hierfür finden sich in US-A-4 536 602, EP-A-101 921 oder DE-A-42 06 992.

Aus EP-A-133 938, EP-A-431 451 und EP-A-132 736 sind Verfahren bekannt, bei denen Bor-, Gallium-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Å definiert sind, eingesetzt werden. Bevorzugt werden metall- oder säuremodifizierte Beta-Zeolithe eingesetzt.

Ein besonderer Nachteil der Zeolithe als Katalysatoren liegt in ihrer aufwendigen Herstellung und damit in ihrem hohem Preis. Die selektive Synthese eines Molekularsiebs (z.B von Zeolithen) durch Hydrothermalsynthese erfordert die genaue Einhaltung vieler Parameter, wie etwa der Kristallisationszeit, der Kristallisationstemperatur, des Druckes oder der Alterungsschritte. Die bei einer Zeolithsynthese üblicherweise eingesetzte strukturdirigierende Verbindung (Templat) muß nach der Kristallisation entfernt werden. Die Entfernung des Templats geschieht in der Regel durch Calcinierung, wobei die organische Verbindung oxidativ abgebaut wird. Aus ökologischen und ökonomischen Gründen ist dies sehr negativ zu bewerten.

Die Herstellung von Kristallen bestimmter Größe oder Morphologie sowie die Präparation geträgerter Zeolithe, welche als Katalysatoren aufgrund verfahrenstechnischer Vorteile oft wünschenswert wäre, ist in der Regel nicht oder nur mit viel Aufwand möglich. Zeolithe besitzen eine sehr enge Porengrößenverteilung. Die Porengrößen variieren je nach Zeolithtyp von 4 bis ca. 12 Å.

Bei einer Zeolith-katalysierten Reaktion haben nur solche Moleküle Zugang zu den katalytisch aktiven Zentren im Inneren des Zeoliths, welche kleiner sind als die Porendimensionen. Reaktanden mit größeren Abmessungen sind vom Inneren der Poren ausgeschlossen.

Für die oben genannte Aminierungsreaktionen bedeutet dies, daß die katalytischen Zentren im Inneren des Zeolithen zur Herstellung von Aminen, welche größer sind als der Porendurchmesser, nicht zur Verfügung stehen.

Der vorliegenden Erfindung lag daher die Aufgabe zugrunde, den zuvor genannten Nachteilen abzuhelfen, insbesondere Katalysatoren für die Aminierung von Olefinen zu finden, deren Präparation deutlich einfacher ist als die der Zeolith-Katalysatoren und eine günstige Porengrößenverteilung auch für voluminösere Aminmoleküle haben.

Der Einsatz von säuremodifizierten Montmorilloniten wird in EP-A-469 719 beschrieben. Die Verwendung von Fällkatalysatoren unter Kombination von zwei oder mehreren Metalloxiden ausgenommen der Kombination Si und Al wird in JP-04082864 dargestellt.

Aus der DE-A-44 31 093 sind oxidische Katalysatoren bekannt, die über den Sol-Gel-Prozess hergestellt werden. Die Bildung von Gelen ist ein metastabiler Vorgang und daher eine Maßstabsvergrößerung sehr schwierig.

Alle Verfahren zur Synthese von Aminen aus Olefinen an nichtzeolithischen Katalysatoren zeichnen sich durch eine geringe Amin-Ausbeute oder geringe Raum-Zeit-Ausbeute aus, oder führen zu einer raschen Desaktivierung der Katalysatoren.

Der vorliegenden Erfindung lag daher die Aufgabe zugrunde, diesen Nachteilen abzuhelfen.

Demgemäß wurde ein neues und verbessertes Verfahren zur Herstellung von Aminen der allgemeinen Formel I 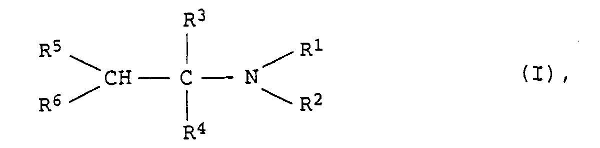 in der
- R¹,R²,R³,R⁴,R⁵,R⁶: Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
- R¹ und R²: gemeinsam eine gesättigte oder ungesättigte C₃-bis C₉-Alkylendikette und
- R³ oder R⁵: C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 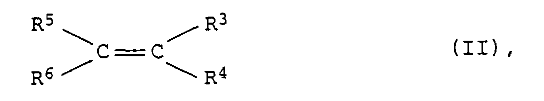 in der R³, R⁴, R⁵ und R⁶ die oben genannten Bedeutungen haben, mit Ammoniak oder primären oder sekundären Aminen der allgemeinen Formel III 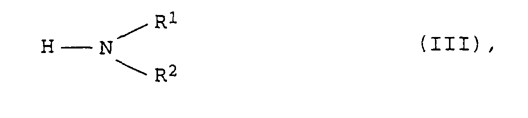 in der R¹ und R² die oben genannten Bedeutungen haben, bei Temperaturen von 200 bis 350°C und einem Druck von 100 bis 300 bar in Gegenwart eines Heterogenkatalysators gefunden, welches dadurch gekennzeichnet ist, daß man als Heterogenkatalysatoren Oxide des Wolframs und/oder des Zirkoniums auf Trägern, wobei weitere Oxide der Gruppen IVB und/oder VIB vorhanden sein können, einsetzt.

Das erfindungsgemäße Verfahren kann wie folgt durchgeführt werden:

Das Olefin II und Ammoniak oder das primäre oder sekundären Amin III kann bei Temperaturen von 200 bis 350°C, bevorzugt 220 bis 330°C, besonders bevorzugt 230 bis 320°C, und Drücken von 100 bis 300 bar, bevorzugt 120 bis 300 bar, besonders bevorzugt 140 bis 290 bar, in Gegenwart von Oxiden des Wolframs und/oder des Zirkoniums auf Trägern, wobei weitere Oxide der Gruppen IVB und/ oder VIB vorhanden sein können, als Katalysator z.B. in einem Druck-Reaktor umsetzt werden, und bevorzugt das erhaltene Amin abgetrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung des Katalysators zurückgedrängt worden. Die Herstellung der Katalysatoren erfolgt in einfacher und gut reproduzierbarer Art und Weise.

Das erfindungsgemäße Verfahren zeichnet sich dadurch aus, daß bereits mit niedrigem Ammoniak- bzw. Amin- Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/ Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Als Katalysatoren für die Aminierung von Olefinen eignen sich Oxide des Wolframs oder des Zirkoniums, wie ZrO₂, WO₂ oder WO₃, oder deren Gemische, wobei weitere Oxide der Gruppen IVB und/ oder VIB vorhanden sein können. Als Trägermaterialien eignen sich Oxide wie SiO₂, Al₂O₃, TiO₂, ZrO₂, MgO, Fe₂O₃, SnO₂, CeO₂ oder Gemische wie SiO₂-Al₂O₃, SiO₂-TiO₂, bevorzugt SiO₂, Al₂O₃, TiO₂, Fe₂O₃, SnO₂, CeO₂ oder Gemische wie SiO₂-Al₂O₃, Carbide oder Nitride wie WC, SiC oder Si₃N₄, Schichtsilikate wie Montmorillonit, Kaolin, Saponit oder Vermiculit sowie Gemische daraus. Bevorzugt sind oxidische Träger, insbesondere solche mit 60 bis 100 Gew.-% SiO₂ und die eine BET-Oberfläche in der Regel von 100 bis 1000 m²g⁻¹, bevorzugt 120 bis 600 m²g⁻¹ nach Aufbringung der Oxide der Gruppen IVB und/oder VIB haben.

Unter den weiteren Oxiden der Gruppen IVB und VIB eignen sich bevorzugt TiO₂, HfO₂, Cr₂O₃, CrO₂, CrO₃, MoO₂ und MoO₃. Die Oxide können entweder als einzelnes Oxid oder als Kombination zweier oder mehrerer Oxide eingesetzt werden, bevorzugt sind Kombinationen zweier Oxide, besonders bevorzugt als Kombination eines Oxides der Gruppe IVB mit einem Oxid der Gruppe VIB. Von dieser Kombination zeichnet sich die folgende besonders aus: ZrO₂-WO₃.

Die Oxidationsstufen der Oxide können sich zwischen der Form wie sie hergestellt wurde und der unter Reaktionsbedingungen vorliegenden unterscheiden. Leicht reduzierbare Metalloxide wie beispielsweise MoO₃ können durch das Reaktionsgemisch aus Ammoniak, Olefinen und Aminen zu niedrigeren Oxidationstufen wie z.B. MoO₂ partiell oder komplett umgewandelt werden. Auch eine der Reaktion vorgelagerte Reduktion kann eingesetzt werden, wenn z.B. die geträgerten Oxide nach einer Kalzinierung in einer hohen Oxidationstufe vorliegen und eine niedrigere gewünscht wird. Als Reduktionsmittel können beispielsweise organische Verbindungen wie Formaldehyd, Oxalsäure, Ameisensäure, i-Propanol oder anorganische Reduktionsmittel wie NaBH₄ oder NH₄HCO₂ (Ammoniumformiat) oder aber auch Wasserstoff eingesetzt werden. Bevorzugt ist Wasserstoff, entweder für sich, oder mit einem Inertgas wie Stickstoff oder Argon verdünnt.

Die Menge der aufgebrachten Oxide der Gruppen IVB und VIB richtet sich nach der jeweiligen Auswahl der gewählten Oxide und des Trägermaterials. Im allgemeinen zeigt sich ein positiver Effekt für die Katalyse bereits bei Mengen von 1 Gew.-% Metall der Gruppe VIB und/oder VIB im fertigen Katalysator, während mehr als 40 Gew.-% in der Regel nur noch geringe Verbesserungen bewirken. Bevorzugt sind daher Mengen von 1 bis 40 Gew.-% der Metalle der Gruppen IVB, VIB oder deren Gemische je Metall, besonders bevorzugt von 5 bis 20 Gew.-% Heterogenkatalysatoren, die 5 bis 15 Gew.-%, bevorzugt 5 bis 10 Gew.-% Wolfram und 8 bis 25 Gew.-%, bevorzugt 10 bis 20 Gew.-% Zirkon auf einem Träger enthalten, sind besonders bevorzugt.

Die Art der Aufbringung der Metalloxide auf die Trägermaterialien kann nach verschiedenen Methoden erfolgen. Aus wirtschaftlichen Gründen wird man meist den Weg einer Tränkung bzw. Imprägnierung wählen, es sind aber auch andere Präparationsmethoden wie beispielsweise Chemical Vapour Deposition möglich. In einer bevorzugten Variante wird der getrocknete und somit wasserfreie Träger mit einer Lösung an Oxidvorläufern versetzt, so daß sich der Oxidvorläufer gleichmäßig auf dem Träger verteilt. Ebenfalls bevorzugt ist eine Aufbringung über eine Sprühimprägnierung, bei der der Träger bewegt mit einer Lösung des zu imprägnierenden Oxidvorläufers besprüht wird, so daß eine gleichmäßige Verteilung der aufgesprühten Lösung resultiert.

Die Art der Lösung kann verschieden sein, z.B. sind alkoholische Lösungen einsetzbar, wiederum aus wirtschaftlichen Gründen wird man allerdings meist wäßrige Lösungen wählen. Als Oxidvorläufer kommen alle Verbindungen der Gruppen IVB und VIB in Frage, die sich nachfolgend in Oxide umwandeln lassen. Diese Umwandlung erfolgt bevorzugt durch eine Kalzinierung. Als derartige Oxidvorläufer sind beispielsweise verschiedene wasserlösliche Salze einsetzbar, wie z.B. TiOSO₄, eine salzsaure Lösung von TiOCl₂ oder TiCl₄, ZrO(NO₃)₂, die wasserlöslichen Salze der Wolframsäure (H₂WO₄), wie sie beispielsweise beim Lösen von Wolframtrioxid in wäßrigem Ammoniak entstehen, also die Monowolframate, und die daraus beim Ansäuern entstehenden Isopolywolframate, z.B. die Parawolframate oder Metawolframate, Ammoniumheptamolybdat oder CrCl₃.

Die Aufbringung kann in einem oder aber auch in mehreren Schritten erfolgen, wenn die Löslichkeit des Oxidvorläufers zu gering ist. Falls mehrmals getränkt wird, wird der Katalysator zwischendurch noch getrocknet oder kalziniert. Falls mehrere Oxide auf dem Träger aufgebracht werden sollen, kann dies in einem oder mehreren Schritten erfolgen. Entscheidend ist hier, ob Bedingungen gefunden werden können, die mit der gleichzeitigen Aufbringung beider Oxide in den gewünschten Mengen vereinbar sind. Falls dies nicht der Fall ist, wird man die beiden Oxide hintereinander aufbringen und eventuell eine Trocknung bzw. Kalzinierung dazwischenschalten. Die Reihenfolge der Aufbringung kann gegebenenfalls Einfluß auf die Katalyse besitzen und sollte daher für die individuelle Kombination von Oxiden optimiert werden.

Falls der gewählte Träger eine hohe Ionenaustauschkapazität besitzt, wie es z.B. bei Schichtsilikaten oder amorphen Alumosilikaten der Fall ist, kann die Aufbringung der Oxidvorläufer auch über einen Ionenaustausch erfolgen. Hierzu bringt man den Träger mit einem Überschuß an Lösung der Oxidvorläufer kontinuierlich oder diskontinuierlich in Kontakt, so daß die austauschfähigen Ionen des Trägermaterials gegen solche der Gruppen IVB und/oder VIB ausgetauscht werden.

Die Kalzinierung der Katalysatorvorläufer nach Aufbringung der Oxidvorläufer auf die Trägermaterialien führt zur Umwandlung der Oxidvorläufer in die katalytisch aktiven Oxide. In Abhängigkeit von der Zeitdauer der Kalzinierung und gewähltem Oxid bzw. Oxidvorläufer liegt die Temperatur bei 250 bis 800°C, vorzugsweise bei 300 bis 600°C.

Die Herstellung der Katalysatoren erfolgt in der Regel durch Tränkung bzw. Imprägnierung von Formkörpern des Trägermaterials. Es ist jedoch ebenfalls möglich, den Träger als Pulver mit den Oxiden oder deren Vorläufern zu beladen und das Material anschließend in einer der üblichen Weisen zu verformen. Für diese Verformung kann ein Bindemittel verwendet werden, anorganische Bindemittel erniedrigen jedoch den Gehalt an Aktivmasse in den fertigen Formkörpern.

Zur Erhöhung der Selektivität, der Standzeit und der Anzahl der möglichen Regenerierungen kann man verschiedene Modifizierungen an den erfindungsgemäßen Oxiden der Gruppen IVB, VIB oder deren Gemischen auf Trägern vornehmen.

Eine Modifizierung der Katalysatoren besteht darin, daß man die unverformten oder die verformten Oxide der Gruppen IVB, VIB oder deren Gemische auf Trägern mit (anderen) Übergangsmetallen wie z.B. Sc, Mn, Fe, Nb, Cu, Zn, Edelmetallen und/oder seltenen Erdmetallen wie z.B. La, Ce oder Y ionenaustauschen bzw. dotieren kann.

Eine vorteilhafte Ausführungsform besteht darin, daß man die verformten erfindungsgemäßen Oxide der Gruppen IVB, VIB oder deren Gemische auf Trägern in einem Strömungsrohr vorlegt und bei 20 bis 100°C z.B. ein Halogenid, ein Acetat, ein Oxalat, ein Citrat oder ein Nitrat der oben beschriebenen Metalle in gelöster Form darüberleitet. Ein derartiger Ionenaustausch kann z.B. an der Wasserstoff-, Ammonium- und Alkaliform der erfindungsgemäßen Oxide der Gruppen IVB, VIB oder deren Gemische auf Trägern vorgenommen werden.

Eine weitere Möglichkeit der Metallaufbringung auf die erfindungsgemäßen Oxide der Gruppen IVB, VIB oder deren Gemische auf Trägern besteht darin, daß man das Material z.B. mit einem Halogenid, einem Nitrat, einem Acetat, einem Oxalat, einem Citrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten Oxiden der Gruppen IVB, VIB oder deren Gemischen auf Trägern kann eine Nachbehandlung mit Wasserstoff und/oder mit Wasserdampf günstig sein.

Eine weitere Möglichkeit der Modifizierung besteht darin, daß man die erfindungsgemäßen Oxide der Gruppen IVB, VIB oder deren Gemische auf Trägern - verformt oder unverformt - einer Behandlung mit Säuren, wie Salzsäure (HCl), Flußsäure (HF), Schwefelsäure (H₂SO₄), Oxalsäure (HO₂C-CO₂H) oder Phosphorsäure (H₃PO₄) unterwirft.

Eine besondere Ausführungsform besteht darin, daß man die erfindungsgemäßen Oxide der Gruppen IVB, VIB oder deren Gemische auf Trägern vor der Verformung mit einer der genannten Säuren 0,001 bis 2 n, bevorzugt 0,05 bis 0,5 n für 1 bis 100 Stunden unter Rückfluß behandelt. Nach Abfiltrieren und Auswaschen wird in der Regel bei 100 bis 160°C getrocknet und bei 200 bis 600°C calciniert. Eine weitere besondere Ausführungsform liegt in einer Säure-Behandlung der erfindungsgemäßen Oxide der Gruppen IVB, VIB oder deren Gemische auf Trägern nach ihrer Verformung mit Bindemittel. Hierbei werden die erfindungsgemäßen Oxide der Gruppen IVB, VIB oder deren Gemische auf Trägern in der Regel 1 bis 3 Stunden zwischen 60 und 80°C mit einer 3 bis 25%igen, insbesondere mit einer 12 bis 20%igen Säure behandelt, anschließend ausgewaschen, bei 100 bis 160°C getrocknet und bei 200 bis 600°C calciniert.

Eine andere Möglichkeit der Modifizierung ist gegeben durch einen Austausch mit Ammonsalzen, z.B. mit NH₄Cl oder mit Mono-, Di- oder Polyaminen. Hierbei wird das verformte Oxid der Gruppen IVB, VIB oder deren Gemische auf Trägern in der Regel bei 60 bis 80°C mit 10 bis 25%iger, bevorzugt 20%iger NH₄Cl-Lösung 2 h kontinuierlich in gewichtsmäßiger Oxid/Ammonchlorid-Lösung von 1:15 ausgetauscht und danach bei 100 bis 120°C getrocknet.

Die Katalysatoren kann man als Stränge mit Durchmessern von z.B. 1 bis 4 mm, Kugeln oder als Tabletten mit z.B. 3 bis 5 mm Durchmesser für die Aminierung der Olefine einsetzen.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
- R¹,R²,R³,R⁴,R⁵,R⁶: - Wasserstoff,
- C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
- C₂- bis C₂₀-Alkenyl, bevorzugt C₂- bis C₁₂-Alkenyl, besonders bevorzugt C₂- bis C₈-Alkenyl wie Vinyl und Allyl,
- C₂- bis C₂₀-Alkinyl, bevorzugt C₂- bis C₈-Alkinyl, insbesondere C₂H und Propargyl,
- C₃- bis C₂₀-Cycloalkyl, bevorzugt C₃- bis C₁₂-Cycloalkyl, besonders bevorzugt C₅- bis C₈-Cycloalkyl wie Cyclopentyl, Cyclohexyl, Cycloheptyl und Cyclooctyl,
- C₄- bis C₂₀-Alkyl-cycloalkyl, bevorzugt C₄- bis C₁₂-Alkylcycloalkyl, besonders bevorzugt C₅- bis C₁₀-Alkyl-cycloalkyl,
- C₄- bis C₂₀-Cycloalkyl-alkyl, bevorzugt C₄- bis C₁₂-Cycloalkyl-alkyl, besonders bevorzugt C₅- bis C₁₀-Cycloalkylalkyl,
- Aryl wie Phenyl, 1-Naphthyl und 2-Naphthyl, bevorzugt Phenyl,
- C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
- C₇- bis C₂₀-Aralkyl, bevorzugt C₇- bis C₁₆-Aralkyl, bevorzugt C₇- bis C₁₂-Phenalkyl wie Phenylmethyl, 1-Phenylethyl, 2-Phenylethyl,
- R¹ und R²: - gemeinsam eine gesättigte oder ungesättigte C₃- bis C₉-Alkylendikette, bevorzugt - (CH₂)₄-, -(CH₂)₅-, -(CH₂)₇- und -CH=CH-CH=CH-,
- R³ oder R⁵: - C₂₁- bis C₂₀₀-Alkyl, bevorzugt C₄₀- bis C₂₀₀-Alkyl, wie Polybutyl, Polyisobutyl, Polypropyl, Polyisopropyl und Polyethyl, besonders bevorzugt Polybutyl und Polyisobutyl,
- C₂₁- bis C₂₀₀-Alkenyl, bevorzugt C₄₀- bis C₂₀₀-Alkenyl, besonders bevorzugt C₇₀- bis C₁₇₀-Alkenyl,
- R³ und R⁵: - gemeinsam eine C₂- bis C₁₂-Alkylendikette, bevorzugt eine C₃-bis C₈-Alkylendikette, besonders bevorzugt -(CH₂)₃-, -(CH₂)₄-, -(CH₂)₅-, -(CH₂)₆- und -(CH₂)₇-, insbesondere -(CH₂)₃- und -(CH₂)₄-.

### Beispiele

### Katalysatorsynthesen

### Katalysator A (Vergleichsbeispiel): SiO₂ (D11-10®)

Katalysator A bestand aus reinen SiO₂-Strängen (4 mm) der BASF (D11-10®). Die BET-Oberfläche betrug 173 m²g⁻¹.

### Katalysator B (Vergleichsbeispiel): SiO₂ (Siliperl®)

Katalysator B bestand aus reinen SiO₂-Kugeln (1,5 bis 3,5 mm) der Fa. Engelhardt (Siliperl® AF125). Die BET-Oberfläche betrug 300 m²g⁻¹.

### Katalysator C: WO₃-ZrO₂/SiO₂

534 g Zirkoncarbonat wurden in 813 g 65%-iger HNO₃ gelöst und innerhalb von 40 min über eine Düse auf 820 g Katalysator B aufgesprüht. Der so imprägnierte Katalysator wurde getrocknet und bei 450°C für 2 Stunden kalziniert. Anschließend wurden 116 g H₂WO₄ in 666 g wäßrigem NH₃ (25%-ig) gelöst innerhalb von 20 min aufgegeben. Der so imprägnierte Katalysator wurde getrocknet und bei 350°C für 2 Stunden kalziniert. Katalysator C enthielt 15,5 Gew.-% Zirkon und 8,4 Gew.-% Wolfram.

### Katalysator D: WO₃-ZrO₂/SiO₂

40 g Katalysator C wurden bei 600°C für 2 Stunden kalziniert. Katalysator D enthielt 12,1 Gew.-% Zirkon und 7,8 Gew.-% Wolfram und besaß eine BET-Oberfläche von 228 m²g⁻¹.

### Katalysator E: WO₃-ZrO₂/SiO₂

577 g Zirkoncarbonat wurden in 800 g 65%iger HNO₃ gelöst und innerhalb von 25 min über eine Düse auf 600 g Katalysator A aufgesprüht. Der so imprägnierte Katalysator wurde getrocknet und bei 450°C für 2 Stunden kalziniert. Anschließend wurden 120 g H₂WO₄ in 690 g wäßrigem NH₃ (25%ig) gelöst und innerhalb von 30 min aufgegeben. Der so imprägnierte Katalysator wurde getrocknet und bei 350°C für 2 Stunden kalziniert. Katalysator E enthielt 15,5 Gew.-% Zirkon und 8,4 Gew.-% Wolfram.

### Katalysator F: ZrO₂/SiO₂

100 g Katalysator A wurden getrocknet und mit einer Lösung aus 2,7 g ZrO(NO₃)₂•H₂O in 100 g dest. Wasser versetzt. Das Wasser wurde langsam i.Vak. abgezogen und anschließend bei 350°C für 5 Stunden kalziniert. Katalysator F enthielt 1 Gew.-% Zirkon und besaß eine BET-Oberfläche von 190 m²g⁻¹.

### Katalysator G: WO₃/SiO₂

100 g Katalysator A wurden getrocknet und mit einer Lösung aus 12,5 g H₂WO₄ in 100 g wäßrigem NH₃ (25%ig) versetzt. Das Wasser wurde langsam i.Vak. abgezogen und anschließend bei 350°C für 5 Stunden kalziniert. Katalysator G enthielt 7,6 Gew.-% Wolfram und besaß eine BET-Oberfläche von 132 m²g⁻¹.

### Katalysator H (Vergleichsbeispiel): Fe₂O₃/SiO₂

100 g Katalysator A wurden getrocknet und mit einer Lösung aus 38 g Fe(NO₃)₃•9H₂O in 95 g dest. Wasser versetzt. Das Wasser wurde langsam i.Vak. abgezogen und anschließend bei 500°C für 2 Stunden kalziniert. Katalysator H enthielt 4,7 Gew.-% Eisen und besaß eine BET-Oberfläche von 181 m²g⁻¹.

### Katalysator I: ZrO₂/SiO₂

100 g Katalysator A wurden getrocknet und mit einer Lösung aus 5,6 g ZrO(NO₃)₂•H₂O in 100 g dest. Wasser versetzt. Das Wasser wurde langsam i.Vak. abgezogen und anschließend bei 500°C für 2 Stunden kalziniert. Katalysator I enthielt 1,9 Gew.-% Zirkon und besaß eine BET-Oberfläche von 180 m²g⁻¹.

### Katalysator J: WO₃-ZrO₂/SiO₂

100 g Katalysator A wurden getrocknet und mit einer Lösung aus 2,7 g ZrO(NO₃)₂•H₂O in 100 g dest. Wasser versetzt. Das Wasser wurde langsam i.Vak. abgezogen und anschließend bei 120°C für 3 Stunden getrocknet. Anschließend wurde mit einer Lösung aus 1,4 g H₂WO₄ in 100 g wäßrigem NH₃ (32%ig) versetzt. Das Wasser wurde langsam i.Vak. abgezogen und bei 500°C für 2 Stunden kalziniert. Katalysator J enthielt 0,9 Gew.-% Zirkon und 1 Gew.-% Wolfram und besaß eine BET-Oberfläche von 138 m²g⁻¹.

### Katalysator K: WO₃/SiO₂

100 g Katalysator A wurden getrocknet und mit einer Lösung aus 25 g H₂WO₄ in 100 g wäßrigem NH₃ (32%ig) versetzt. Das Wasser wurde langsam i.Vak. abgezogen und anschließend bei 500°C für 2 Stunden kalziniert. Katalysator K enthielt 14,5 Gew.-% Wolfram und besaß eine BET-Oberfläche von 107 m²g⁻¹.

### Katalysator L: ZrO₂/SiO₂

75 g Katalysator A wurden getrocknet und mit einer Lösung aus 20,5 g ZrO(NO₃)₂•H₂O in 75 g dest. Wasser versetzt. Das Wasser wurde langsam i.Vak. abgezogen und anschließend bei 350°C für 5 Stunden kalziniert. Katalysator L enthielt 8,6 Gew.-% Zirkon.

### Aminierungsbeispiele

Die Versuche wurden in einem Rohrreaktor (6 mm Innendurchmesser) unter isothermen Bedingungen bei 260 bis 300°C und einem Druck von 280 bar mit einem Gemisch aus Isobuten und Ammoniak im molaren Verhältnis von 1:1,5 durchgeführt. Die Reaktionsprodukte wurden im Gaschromatographen analysiert.

Die Ergebnisse sind in Tabelle 1 zusammengestellt und zeigen, daß die erfindungsgemäßen Katalysatoren deutlich höhere Ausbeuten als die reinen Trägermaterialien besitzen. Andere Oxide zeigen geringe Aktivität.

**Tabelle 1**

| tert.-Butylamin (NH₃: C₄H₈ = 1,5) | | | | | | |
|---|---|---|---|---|---|---|
| Katalysator | Druck | Temperatur | tert.-Butylamin-Ausbeute [Gew.-%] | | | Litergewicht |
| Nr. | [bar] | [°C] | WHSV 0,7 [g/g·h] | WHSV 1,5 [g/g·h] | WHSV 3 [g/g·h] | [kg/l] |
| A | 280 | 300 | 2,01 | 0,92 | 0,52 | 0,37 |
| B | 280 | 300 | 3,35 | 1,76 | 0,88 | 0,41 |
| C | 280 | 300 | 13,47 | 10,42 | 7,30 | 0,54 |
| D | 280 | 300 | 9,84 | 7,93 | 4,45 | 0,58 |
| E | 280 | 300 | 14,07 | 11,78 | 7,26 | 0,53 |
| F | 280 | 300 | 7,50 | 3,83 | 2,01 | 0,38 |
| G | 280 | 300 | 11,64 | 7,15 | 4,03 | 0,38 |
| H | 280 | 300 | 1,19 | 0,26 | | 0,39 |
| I | 280 | 300 | 5,00 | 2,54 | 1,35 | 0,38 |
| J | 280 | 300 | 6,02 | 3,42 | | 0,38 |
| K | 280 | 300 | 8,97 | 5,38 | 3,68 | 0,40 |
| L | 280 | 300 | 5,34 | 3,42 | 2,28 | 0,40 |

## Patentansprüche

1. Verfahren zur Herstellung von Aminen der allgemeinen Formel I 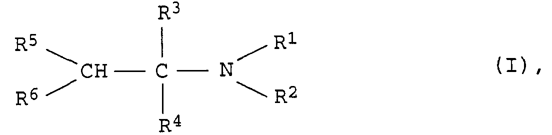 in der
R¹,R²,R³,R⁴,R⁵,R⁶ Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
R¹ und R² gemeinsam eine gesättigte oder ungesättigte C₃- bis C₉-Alkylendikette und
R³ oder R⁵ C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 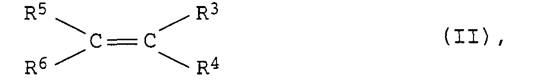 in der R³, R⁴, R⁵ und R⁶ die oben genannten Bedeutungen haben, mit Ammoniak oder primären oder sekundären Aminen der allgemeinen Formel III 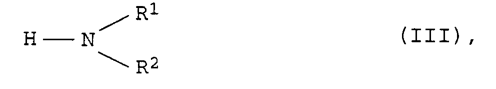 in der R¹ und R² die oben genannten Bedeutungen haben, bei Temperaturen von 200 bis 350°C und einem Druck von 100 bis 300 bar in Gegenwart eines Heterogenkatalysators, **dadurch gekennzeichnet, daß** man als Heterogenkatalysatoren Oxide des Wolframs und/oder des Zirkoniums auf Trägern, wobei weitere Oxide der Gruppen IVB und/oder VIB vorhanden sein können, einsetzt.

2. Verfahren zur Herstellung von Aminen I nach Anspruch 1, **dadurch gekennzeichnet, daß** man das gebildete Amin I abtrennt und die nichtumgesetzten Einsatzstoffe II und III zurückführt.

3. Verfahren zur Herstellung von Aminen nach den Ansprüchen 1 bis 2, **dadurch gekennzeichnet, daß** man als Olefin II Isobuten, Diisobuten, Cyclopenten, Cyclohexen oder Polyisobuten einsetzt.

4. Verfahren zur Herstellung von Aminen nach den Ansprüchen 1 bis 3, **dadurch gekennzeichnet, daß** man als Oxide des Wolframs WO₂, WO₃, als Oxide des Zirkoniums ZrO₂ und als weitere Oxide der Gruppen IVB und VIB TiO₂, HfO₂, Cr₂O₃, CrO₂, CrO₃, MoO₂, MoO₃ oder deren Gemische einsetzt.

5. Verfahren zur Herstellung von Aminen nach den Ansprüchen 1 bis 4, **dadurch gekennzeichnet, daß** man WO₃, ZrO₂ oder deren Gemische auf einem Träger einsetzt.

6. Verfahren zur Herstellung von Aminen nach den Ansprüchen 1 bis 5, **dadurch gekennzeichnet, daß** man Heterogenkatalysatoren einsetzt, die 5 bis 15 Gew.-% Wolfram und 8 bis 25 Gew.-% Zirkon auf einem Träger enthalten.

7. Verfahren zur Herstellung von Aminen nach den Ansprüchen 1 bis 6, **dadurch gekennzeichnet, daß** man Heterogenkatalysatoren einsetzt, die im wesentlichen frei von Alkali- oder Erdalkali-Ionen sind.

8. Verfahren zur Herstellung von Aminen nach den Ansprüchen 1 bis 7, **dadurch gekennzeichnet, daß** man Heterogenkatalysatoren einsetzt, die zusätzlich mit einem oder mehreren Übergangsmetallen und/oder mit einem oder mehreren Elementen der Seltenen Erden dotiert sind.

9. Verfahren zur Herstellung von Aminen nach den Ansprüchen 1 bis 8, **dadurch gekennzeichnet, daß** der Träger zu 60 bis 100 Gew.-% aus Siliziumdioxid besteht.

10. Verfahren zur Herstellung von Aminen nach den Ansprüchen 1 bis 9, **dadurch gekennzeichnet, daß** man Heterogenkatalysatoren mit einer BET-Oberfläche von 100 bis 1000 m²g⁻¹ einsetzt.

11. Verfahren zur Herstellung von Aminen nach den Ansprüchen 1 bis 10, **dadurch gekennzeichnet, daß** man Heterogenkatalysatoren mit einer BET-Oberfläche von 120 bis 600 m²g⁻¹ einsetzt.

## Claims

1. A process for the preparation of amines of the formula I 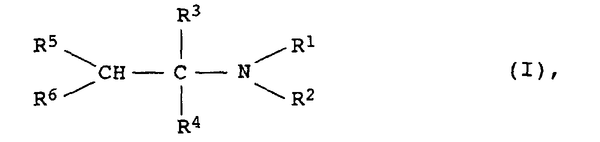 where
R¹, R², R³, R⁴, R⁵ and R⁶ are each hydrogen, C₁-C₂₀-alkyl, C₂-C₂₀-alkenyl, C₂-C₂₀-alkynyl, C₃-C₂₀-cycloalkyl, C₄-C₂₀-alkylcycloalkyl, C₄-C₂₀-cycloalkylalkyl, aryl, C₇-C₂₀-alkylaryl or C₇-C₂₀-aralkyl,
R¹ and R² together form a saturated or unsaturated C₃-C₉-alkylene chain and
R³ or R⁵ is C₂₁-C₂₀₀-alkyl or C₂₁-C₂₀₀-alkenyl, or R³ and R⁵ together form a C₂-C₁₂-alkylene chain,
by reacting an olefin of the formula II 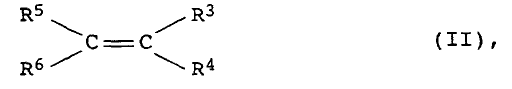 where R³, R⁴, R⁵ and R⁶ have the abovementioned meanings, with ammonia or a primary or secondary amine of the formula III 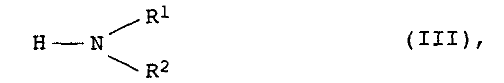 where R¹ and R² have the abovementioned meanings, at from 200 to 350°C and from 100 to 300 bar in the presence of a heterogeneous catalyst, wherein the heterogeneous catalyst used is an oxide of tungsten and/or of zirconium on a carrier, with or without further oxides of groups IVB and/or VIB being present.

2. A process as claimed in claim 1 for the preparation of amines I, wherein the amine I formed is separated off and the unconverted starting materials II and III are recycled.

3. A process as claimed in claim 1 or 2 for the preparation of amines, wherein the olefin II used is isobutene, diisobutene, cyclopentene, cyclohexene or polyisobutene.

4. A process as claimed in any of claims 1 to 3 for the preparation of amines, wherein WO₂ or WO₃ is used as an oxide of tungsten, ZrO₂ is used as an oxide of zirconium, and TiO₂, HfO₂, Cr₂O₃, CrO₂, CrO₃, MoO₂, MoO₃ or a mixture thereof is used as further oxides of groups IVB and VIB.

5. A process as claimed in any of claims 1 to 4 for the preparation of amines, wherein WO₃, ZrO₂ or a mixture thereof on a carrier is used.

6. A process as claimed in any of claims 1 to 5 for the preparation of amines, wherein a heterogeneous catalyst which contains from 5 to 15% by weight of tungsten and from 8 to 25% by weight of zirconium on a carrier is used.

7. A process as claimed in any of claims 1 to 6 for the preparation of amines, wherein heterogeneous catalysts which are essentially free of alkali metal ions or alkaline earth metal ions are used.

8. A process as claimed in any of claims 1 to 7 for the preparation of amines, wherein heterogeneous catalysts which are additionally doped with one or more transition metals or with one or more rare earth elements are used.

9. A process as claimed in any of claims 1 to 8 for the preparation of amines, wherein the carrier comprises from 60 to 100% by weight of silica.

10. A process as claimed in any of claims 1 to 9 for the preparation of amines, wherein a heterogeneous catalyst having a BET surface area of from 100 to 1000 m²g⁻¹ is used.

11. A process as claimed in any of claims 1 to 10 for the preparation of amines, wherein a heterogeneous catalyst having a BET surface area of from 120 to 600 m²g⁻¹ is used.

## Revendications

1. Procédé de préparation d'amines de formule générale I 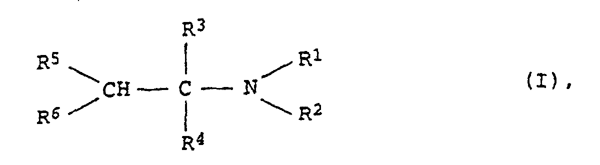 dans laquelle
R¹, R², R³, R⁴, R⁵, R⁶ représentent un atome d'hydrogène, un groupe alkyle en C₁ à C₂₀, alcényle en C₂ à C₂₀, alcynyle en C₂ à C₂₀, cycloalkyle en C₃ à C₂₀, (alkyle en C₄ à C₂₀)-cycloalkyle, (cycloalkyle en C₄ à C₂₀)-alkyle, aryle, (alkyle en C₇ à C₂₀)-aryle ou aralkyle en C₇ à C₂₀,
R¹ et R² représentent ensemble une double chaîne alkylène en C₃ à C₉ saturée ou insaturée et
R³ ou R⁵ représente un groupe alkyle en C₂₁ à C₂₀₀, alcényle en C₂₁ à C₂₀₀ ou ensemble une double chaîne alkylène en C₂ à C₁₂,
par réaction d'oléfines de formule générale II 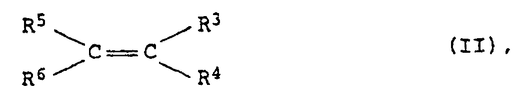 dans laquelle R³, R⁴, R⁵ et R⁶ prennent les significations précitées, avec de l'ammoniac ou des amines primaires ou secondaires de formule générale III 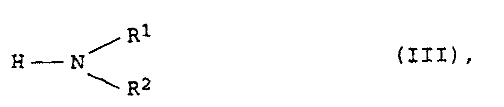 dans laquelle R¹ et R² prennent les significations précitées, à des températures de 200 à 350°C et sous une pression de 100 à 300 bars en présence d'un catalyseur hétérogène, **caractérisé en ce que** l'on met en oeuvre en tant que catalyseur hétérogène, des oxydes de tungstène et/ou de zirconium sur des supports, d'autres oxydes des Groupes IVB et/ou VIB pouvant être présents.

2. Procédé de préparation d'amines I selon la revendication 1, **caractérisé en ce que** l'on sépare l'amine formée I et on recycle les matières mises en oeuvre, II et III, n'ayant pas réagi.

3. Procédé de préparation d'amines selon les revendications 1 à 2, **caractérisé en ce que** l'on met en oeuvre en tant qu'oléfine II, l'isobutène, le diisobutène, le cyclopentène, le cyclohexène ou le polyisobutène.

4. Procédé de préparation d'amines selon les revendications 1 à 3, **caractérisé en ce que** l'on met en oeuvre en tant qu'oxydes de tungstène, WO₂, WO₃, en tant qu'oxydes de zirconium, ZrO₂ et en tant qu'oxydes supplémentaires des Groupes IVB et VIB, TiO₂, HfO₂, Cr₂O₃, CrO₂, CrO₃, MoO₂, MoO₃ ou leurs mélanges.

5. Procédé de préparation d'amines selon les revendications 1 à 4, **caractérisé en ce que** l'on met en oeuvre WO₃, ZrO₂ ou leurs mélanges sur un support.

6. Procédé de préparation d'amines selon les revendications 1 à 5, **caractérisé en ce que** l'on met en oeuvre des catalyseurs hétérogènes qui contiennent 5 à 15% en poids de tungstène et 8 à 25% en poids de zircon sur un support.

7. Procédé de préparation d'amines selon les revendications 1 à 6, **caractérisé en ce que** l'on met en oeuvre des catalyseurs hétérogènes qui sont essentiellement exempts d'ions de métal alcalin ou alcalino-terreux.

8. Procédé de préparation d'amines selon les revendications 1 à 7, **caractérisé en ce que** l'on met en oeuvre des catalyseurs hétérogènes, qui sont dopés en plus avec un ou plusieurs métaux de transition et/ou avec un ou plusieurs éléments des terres rares.

9. Procédé de préparation d'amines selon les revendications 1 à 8, **caractérisé en ce que**, le support est constitué de dioxyde de silicium à raison de 60 à 100% en poids.

10. Procédé de préparation d'amines selon les revendications 1 à 9, **caractérisé en ce que** l'on met en oeuvre des catalyseurs hétérogènes ayant une surface spécifique BET de 100 à 1 000 m²g⁻¹.

11. Procédé de préparation d'amines selon les revendications 1 à 10, **caractérisé en ce que** l'on met en oeuvre des catalyseurs hétérogènes ayant une surface spécifique BET de 120 à 600 m²g⁻¹.
